# FASCICULE DE BREVET EUROPEEN

(11) **EP 0 477 107 B1**
(45) Date de publication et mention de la délivrance du brevet: **07.01.1998**
(21) Numéro de dépôt: 91402511.9
(22) Date de dépôt: 20.09.1991
(51) Int. Cl.: A61K 47/48

(54) **Stéroides inclus dans des cyclodextrines**
In Cyclodextrinen eingeschlossene Steroide
Steroids contained in cyclodextrins

(30) Priorité: 21.09.1990 FR 9011687
(43) Date de publication de la demande: 25.03.1992
(73) Titulaire: Laboratoires BESINS ISCOVESCO Société anonyme dite :, 75003 Paris (FR)
(72) Inventeur: Agnus, Benoît, F-94210 La Varenne Saint Hilaire (FR); Duchene, Dominique, F-75116 Paris (FR); Wouessidjewe, Denis, F-92160 Antony (FR); Sebille, Bernard, F-92140 Clamart (FR)
(74) Mandataire: Bruder, Michel

(56) Documents cités:
- EP-A- 0 399 716
- WO-A-90/01320
- US-A- 4 383 992

## Description

La présente invention a trait à des inclusions de stéroïdes à noyau A saturé et de leurs métabolites dans les cyclodextrines, inclusions qui peuvent présenter la forme de complexes et qui s'appliquent en particulier à des stéroïdes sexuels endogènes. Elle concerne également un procédé pour leur fabrication.

Les stéroïdes ont généralement une très faible solubilité dans l'eau, ce qui rend leur utilisation très difficile, sinon impossible dans de nombreux cas, par exemple pour leur administration par voie intraveineuse. Il est connu d'augmenter la solubilité des stéroïdes en ajoutant des agents solubilisants tels que la lécithine, mais cette technique est peu satisfaisante en raison de l'instabilité chimique qui en résulte.

On remarquera également que, toujours en raison de leur mauvaise solubilité dans l'eau, les stéroïdes administrés par voie orale ont une mauvaise biodisponibilité et un faible taux de dissolution.

Il est connu que, lorsqu'on dissous, dans une solution aqueuse de cyclodextrine, une autre substance dont les molécules sont au moins partiellement apolaires, cette fraction moléculaire apolaire présente une tendance à s'insérer dans les cavités de la cyclodextrine également apolaires. Mais il est bien connu que les complexes d'inclusion ainsi obtenus sont beaucoup plus difficilement solubles dans l'eau que la cyclodextrine libre elle-même (voir Chem.Berichte, 90,2561-2573 (1957)). Mais, comme la cyclodextrine elle-même ne présente qu'une solubilité faible à température ordinaire, les complexes ainsi formés ont tendance à se séparer des solutions sous formes cristallines d'où la quasi impossibilité de les administrer oralement et plus encore par injection.

On a constaté, par ailleurs, que les stéroïdes à noyaux A et/ou B étaient souvent difficiles à loger et à complexer. Ceci est caractéristique dans le cas du brevet américain N°4 383 992 (LIPARI) où un certificat de correction restreint la portée du brevet à des stéroïdes présentant des insaturations sur les noyaux A et B.

L'utilisation de diméthyl-béta-cyclodextrine proposée par le brevet français N°2 484 252 permet d'augmenter la solubilité de composés tels que la nortestostérone, l'hydrocortisone, l'androstène-dione, l'oestrone, la méthyltestostérone ou la progestérone, tous ces composés étant des stéroïdes dont le noyau A est insaturé; ce procédé ne semble pas avoir donné de résultats satisfaisants avec d'autres types de stéroïdes et en particulier il ne semble pas avoir pu être appliqué à des stéroïdes dont le noyau A est saturé. Il en va de même avec la solution utilisée par le brevet américain N°4 877 774 (PITHA) qui utilise de la gammacyclodextrine pour complexer de la testostérone, de l'oestradiol ou de la progestérone caractérisés par leurs noyaux A insaturés. Les mêmes remarques s'imposent pour le brevet américain N°326 196 (AKZO) ou encore le brevet belge N°894 825 (RICHTER Gédéon) qui concerne également des inclusions, dans une gammacyclodextrine, de méthyltestostérone, de spironolactone, d'hydrocortisone, de prednisolone, dexaméthasone et de triamcinolone, stéroïdes présentant tous un noyau A insaturé.

On peut donc considérer que les techniques antérieures étaient inapplicables à des stéroïdes à noyau A saturé et estimer qu'actuellement, il n'existe pas de solution réellement satisfaisante pour rendre suffisamment solubles des stéroïdes à noyau A saturé, par exemple pour les administrer par voie orale et plus encore par injection et notamment par voie intraveineuse.

La présente invention concerne donc plus particulièrement la mise en solution de stéroïdes comprenant de 18 à 27 atomes de carbone et présentant un noyau A saturé. Par ailleurs, comme on le verra ci-après, la présente invention s'applique tout particulièrement aux stéroïdes de ce type présentant :
- au moins un groupe méthyle en position 10 et/ou 13
- éventuellement un autre radical méthyle en position 1 ou 2,
- en position 17, l'hydrogène et(ou un radical hydrocarbure linéaire ou ramifié,
- éventuellement en positions 3,7,11,12,14,17 ou sur ladite chaîne hydrocarbure, au moins une fonction alcool et/ou cétone et/ou acide.
- éventuellement un radical acide en position 2.
- éventuellement un hétérocycle nitré entre les positions 2 et 3.

Il convient de rappeler à ce sujet que les stéroïdes présentent comme structure de base celle du perhydrocyclopentanophénanthrène 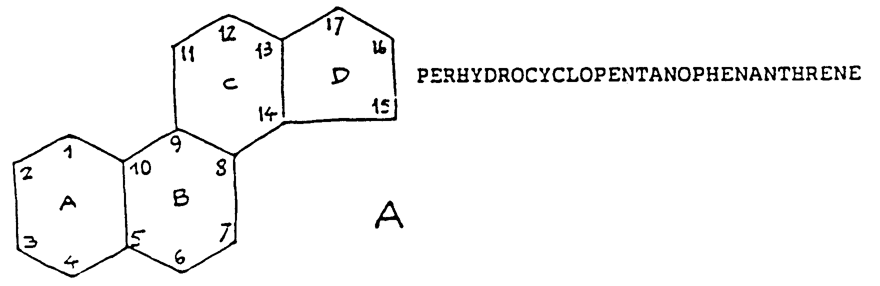 qui est le dérivé saturé du cyclo(a) phénanthrène

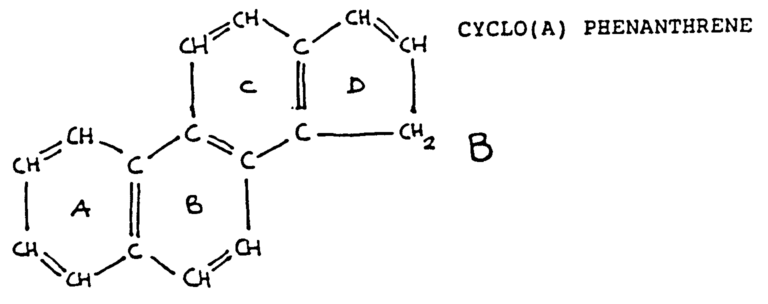

Les repères numériques des positions sont portés sur la formule A.

Dans la plupart des cas, les cycles de ces molécules de type cyclohexane sont conformés en "chaises de pont" c'est-à-dire qu'ils présentent des plis extrêmes opposés par rapport à la partie centrale, comme on le voit sur les formules suivantes :

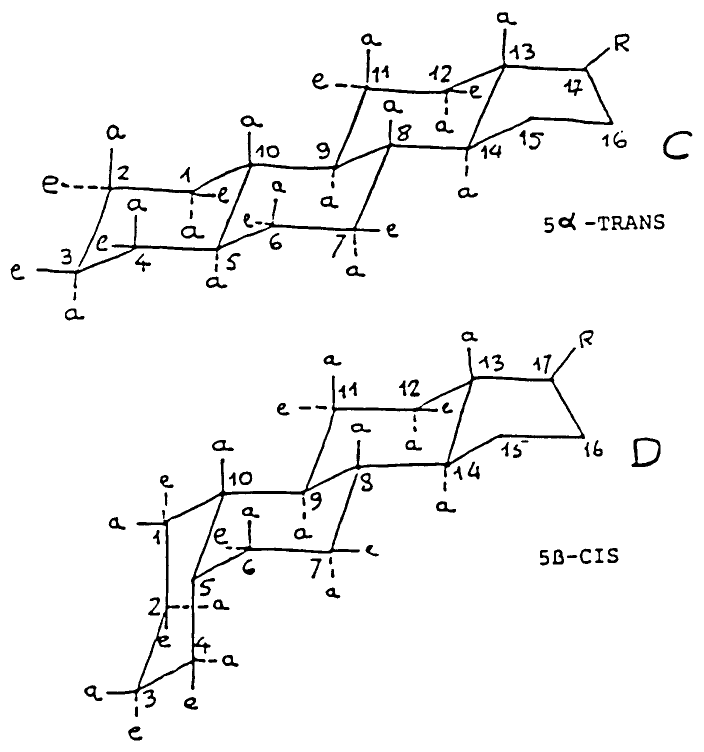

Selon l'orientation du noyau cyclohexane A extrême (à gauche sur les formules) par rapport aux autres cycles de la molécule, on distingue les formes 5alpha-trans(formule C) qui correspondent aux séries allo et les formes 5béta-cis (formule D) qui correspondent aux séries normales.

On a également représenté sur ces formules C et D l'orientation des substitués axiaux (a) et équatoriaux (e). Sur un carbone donné, la liaison (a) et (e) la plus proche de l'observateur est généralement représentée en traits pleins (béta) et l'autre en pointillés (alpha). Si l'orientation est inconnue, on la représente en zigzag (xi).

Dans les stéroïdes naturels, les cycles B et C sont toujours en position respective trans et les cycles C et D presque toujours également en position respective trans.

La présente invention concerne les stéroïdes à cycle A saturé, ce qui exclut, par exemple, l'oestrone ou hydroxy-3-oestratriène 1,3,5(10)-one-17, 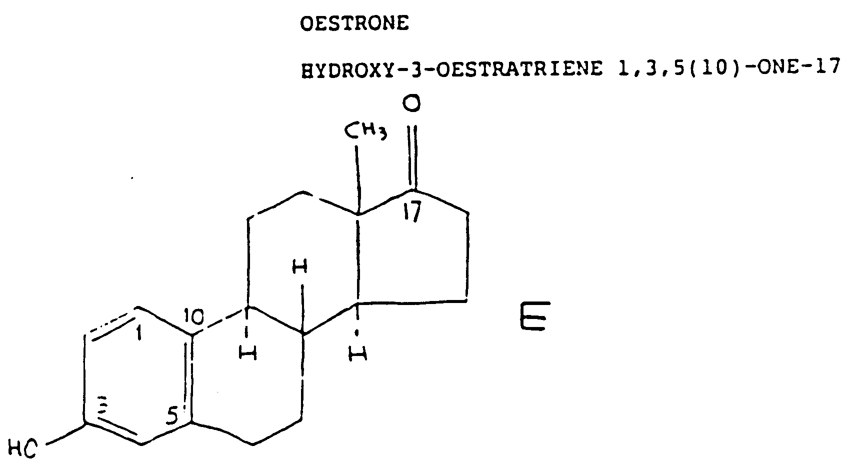 mais inclut en particulier les trois composés suivants que l'on retrouvera dans les exemples :
1) la prégnanol-3alpha-one-20. 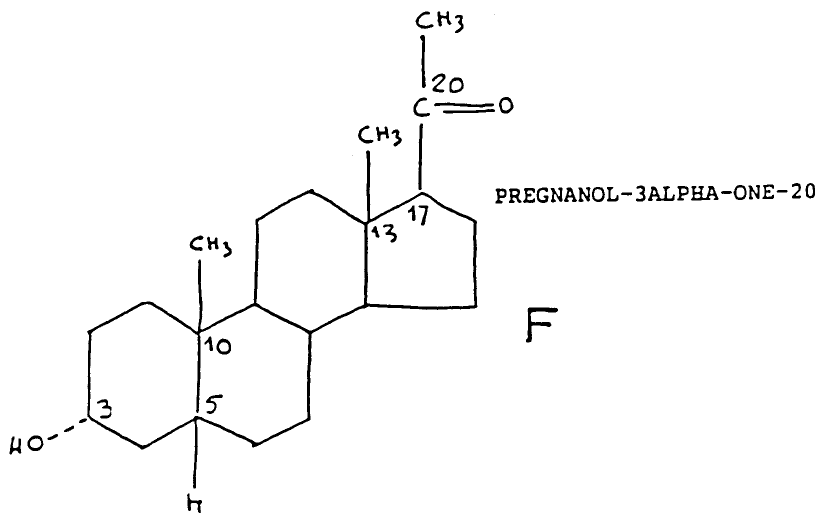
2) la dihydro-4-progestérone ou prégnane-diol-3,20 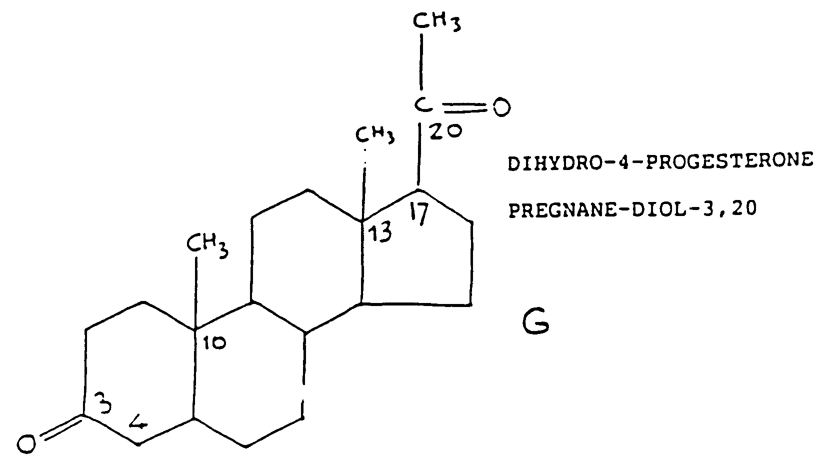
3) la dihydro-4-testotérone ou stanolone ou encore hydroxy-17β-androstane-5alpha-one-3

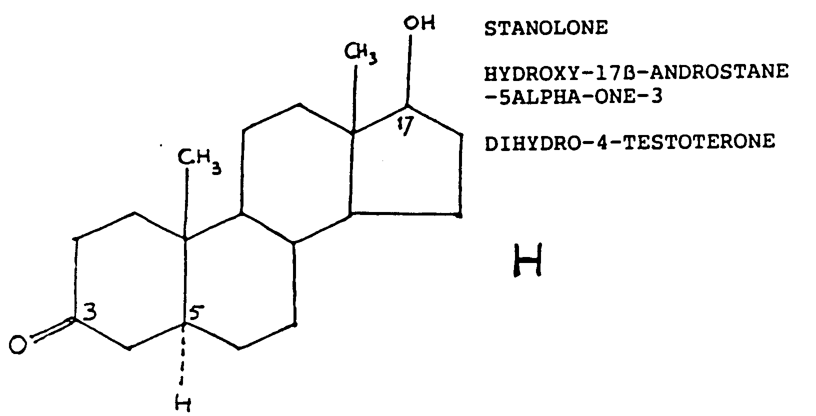

Les stéroïdes visés par l'invention présentent de 18 à 27 atomes de carbone et répondent à la formule générale 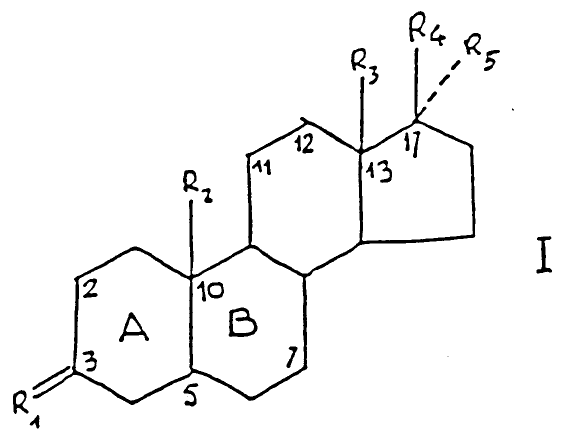 dans laquelle :
- les noyaux A et B sont saturés,
   R₁ est de l'hydrogène et/ou un radical alcool (-OH) ou cétone (=OH);
- l'un au plus de R₂ et R₃ est un hydrogène, au moins l'un de R₂ et R₃ étant un radical méthyle;
- R₄ est un hydrogène, un alcool ou une chaîne hydrocarbure aliphatique éventuellement ramifiée et portant éventuellement une ou plusieurs fonctions alcool et/ou cétone et/ou acide;
- R₅ est un atome d'hydrogène ou un radical alcool ou un hydrocarbure aliphatique.
- Dans la formule ci-dessus R₄ et/ou R₅ peuvent être un radical cétone ; un hétérocycle éventuellement aminé peut être formé entre les positions 2 et 3 en incluant les deux atomes de carbone correspondants et un ou plusieurs atomes d'azote et éventuellement d'oxygène. Par ailleurs un radical méthyle peut être fixé en positon 1 ou 2, un radical acide peut être fixé en position 2, et une double liaison peut exister entre les positions 5 et 6. Une fonction cétone, et/ou une fonction alcool et/ou un hydrogène peuvent être fixés en positions 7 et/ou 11 et/ou 12.

Les principaux stéroïdes répondant à la définition qui vient d'être donnée sont présentés dans le tableau suivant avec indication des différents radicaux ou fonctions selon les positions numérotées en tête du tableau.

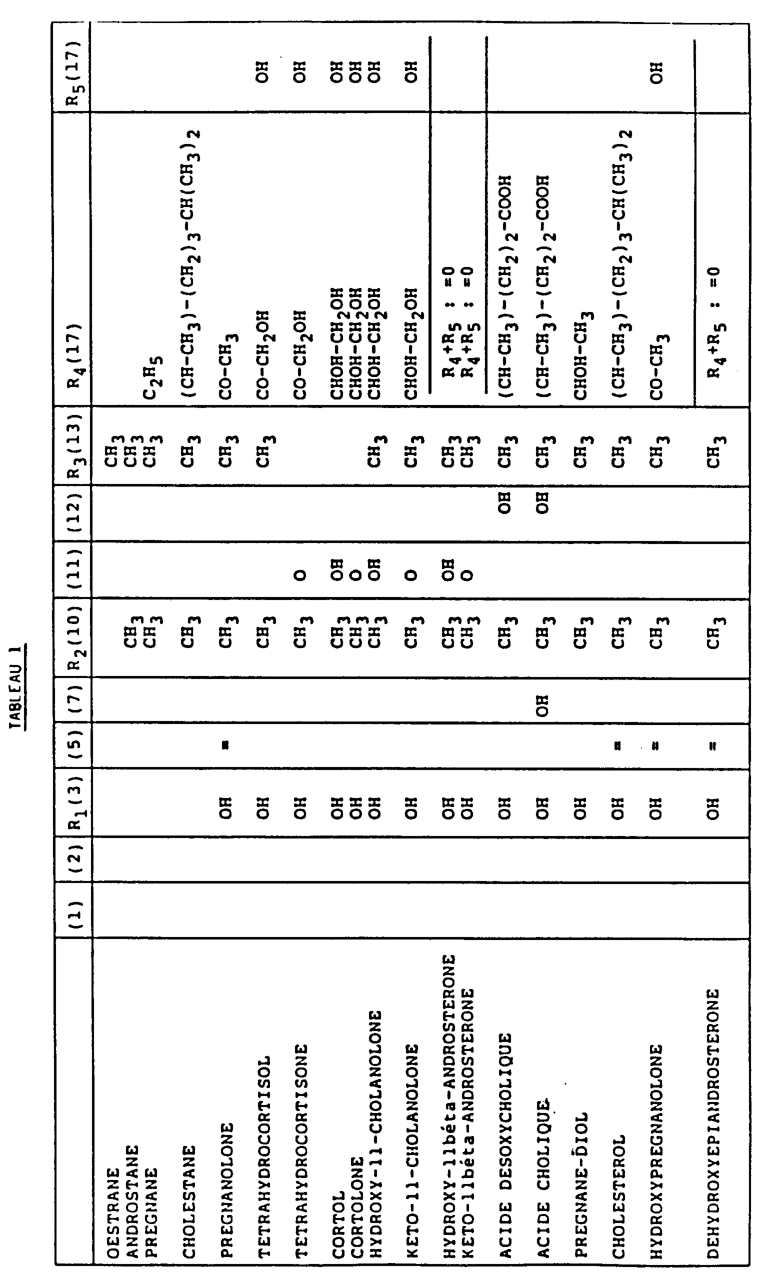

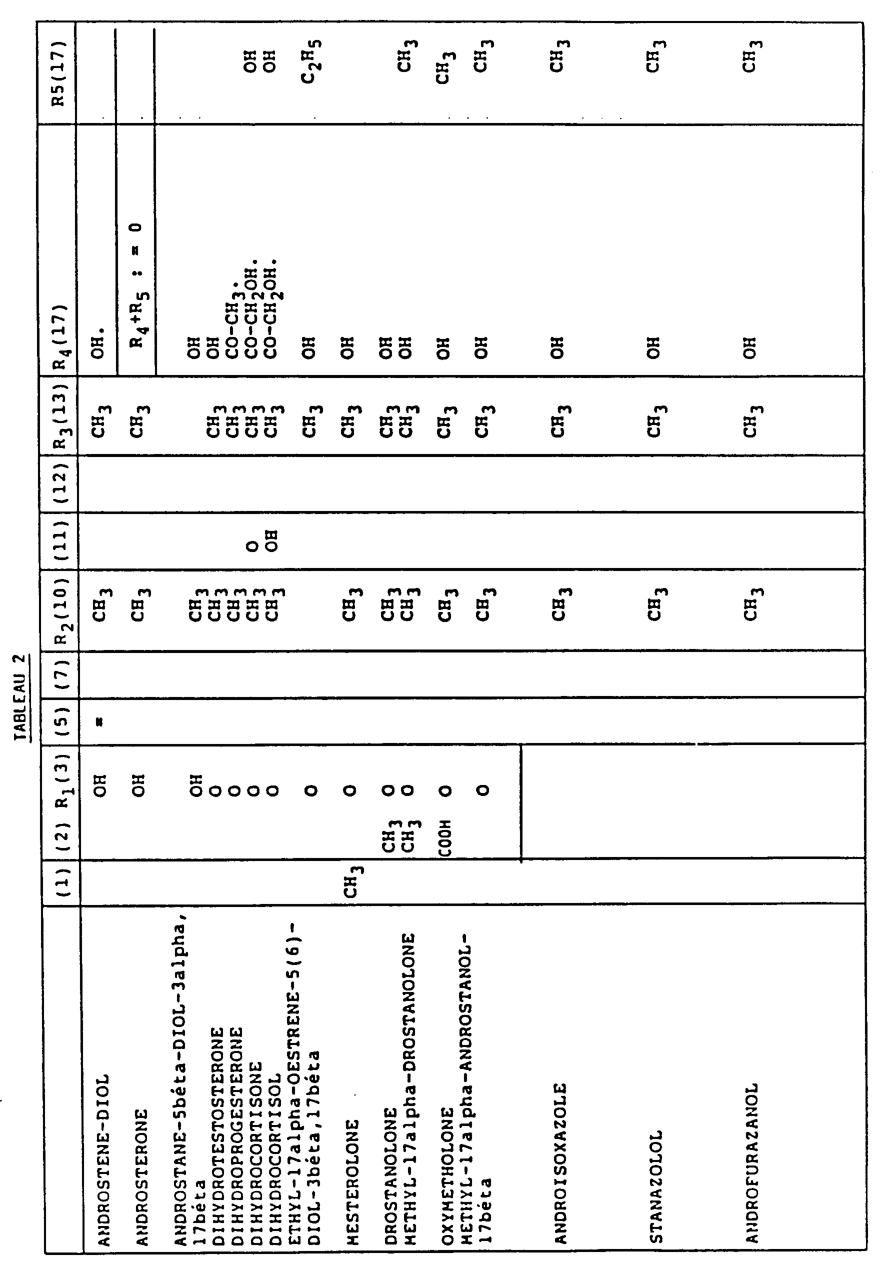

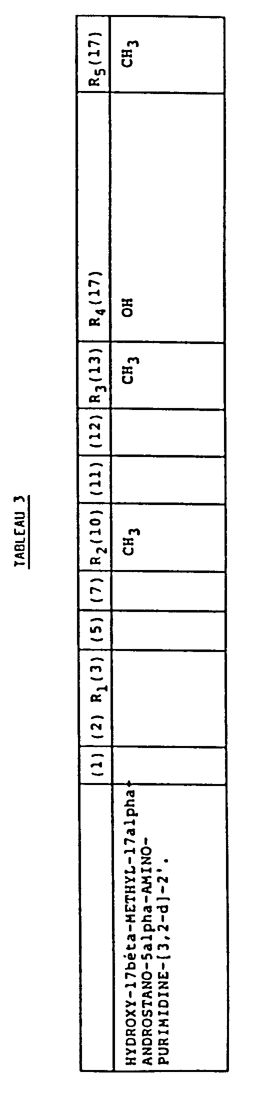

En ce qui concerne les cyclodextrines, on rappellera qu'il s'agit d'oligosaccharides cycliques composés généralement de 6 à 8 unités de glucoses (alpha, béta, gamma cyclodextrine) reliés entre eux par une liaison covalente alpha α 1-4.

Les types de cyclodextrines auxquels fait appel la présente invention sont notamment la bétacyclodextrine, la gammacyclodextrine et l'hydroxypropylbétacyclodextrine, en tout ou partie substituée. Les cyclodextrines sont caractérisées par le fait que la partie intérieure des molécules est apolaire, tandis que la partie extérieure est polaire. Les stéroïdes en solution aqueuse (ou hydroalcoolique dans un rapport judicieusement choisi) s'incluent dans la cyclodextrine par interaction hydrophobe, que l'on peut assimiler dans une certaine mesure à une microencapsulation. L'expérience amène à considérer que les inclusions obtenues sont des complexes qui constituent une forme galénique; on en vient actuellement à considérer le complexe principe actif/bétacyclodextrine comme une entité propre ; les propriétés physiques de la molécule deviennent différentes dans la molécule complexée. De ce fait, on peut donner le nom "d'excipient actif" à la cyclodextrine.

En ce qui concerne l'inclusion elle-même dans les types de cyclodextrine précédemment cités, l'expérience a montré que les stéroïdes à noyau A saturé et en particulier les stéroïdes du type 5β ci-dessus représenté (stéroïdes sexuels endogènes et autres) permettent d'obtenir des résultats particulièrement significatifs.

Pour mieux faire comprendre les caractéristiques techniques et les avantages de la présente invention, on va décrire des exemples de réalisation étant bien entendu que ceux-ci ne sont pas limitatifs quant à leurs modes de mise en oeuvre et aux applications qu'on peut en faire.

On se référera aux figures suivantes qui représentent schématiquement des courbes de solubilité:
La figure 1, la solubilité de la prégnane 5-béta-ole-3-alpha-one-20 ou prégnanolone (en mg/l) en fonction de la concentration de la bétacyclodextrine (en 10-³M) dans l'eau ;
la figure 2, la solubilité de cette même prégnanolone en fonction de la concentration de l'hydroxypropylcyclodextrine dans l'eau (mêmes unités que pour la figure 1) ;
la figure 3, la solubilité de la dihydro-4-testostérone (en mg/l) en fonction de la concentration de la bétacyclodextrine dans l'eau (en 10-³M) ;
la figure 4, la solubilité de cette même dihydro-testostérone (en mg/l) en fonction de la concentration de l'hydroxypropylbétacyclodextrine dans l'eau (en 10-³M) ;
la figure 5, la solubilité de la dihydro-4-progestérone (en mg/l) en fonction de la concentration de la bétacyclodextrine dans l'eau (en 10-³M) ;
la figure 6, la solubilité de la dihydro-4-progestérone (en mg/l) en fonction de la concentration de l'hydroxypropylbétacyclodextrine dans l'eau (en 10-³M) ;
la figure 7, la solubilité de la prégnane-5-béta-ole-3-alpha-one-20 (en %) en fonction du temps dans divers cas comparés ;
la figure 8, la solubilité de la dihydro-4-testostérone (en %) en fonction du temps, dans divers cas comparés ;

### EXEMPLE 1

### SOLUBILITE DE LA PREGNANE 5-BETA-OL-3-ALPHA-ONE-20 EN FONCTION DE LA CONCENTRATION DE BETACYCLO-DEXTRINE DANS L'EAU.

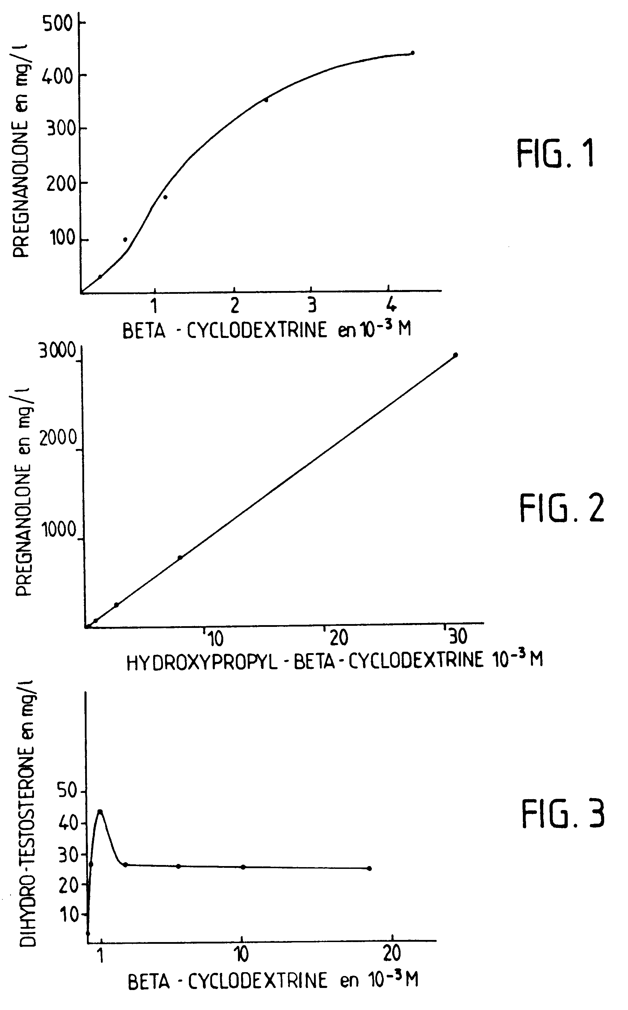

On réalise des solutions de bétacyclodextrines à différentes concentrations de 0 à 5mM. A ces différentes solutions, on ajoute de la prégnane-5-béta-ol-3alpha-one-20 par pincées et ce jusqu'à apparition d'un précipité. On met sous agitation magnétique à 25° pendant 72 heures en prenant soin de vérifier à intervalles réguliers qu'il y a toujours saturation en stéroïdes. Au bout de 72 heures, on filtre les solutions ainsi réalisées et on dose le surnageant. Les résultats obtenus font l'objet de la courbe de la figure 1.

### EXEMPLE 2

### SOLUBILITE DE LA PREGNANE-5BETA-OLE-3ALPHA-ONE 20 EN FONCTION DE LA CONCENTRATION D'HYDROXYPROPYL-BETACYCLODEXTRINE DANS L'EAU.

Excepté la concentration en ladite cyclodextrine qui varie de 0 à 32mM, on procède selon le même protocole qu'à l'exemple 1 ; les résultats sont illustrés par la courbe de la figure 2.

### EXEMPLE 3

### SOLUBILITE DU DIHYDRO-4-TESTOSTERONE EN FONCTION DE LA CONCENTRATION EN BETACYCLODEXTRINE DANS L'EAU.

On procède selon le même protocole qu'à l'exemple 1. Les résultats sont représentés par la courbe de la figure 3.

### EXEMPLE 4

### SOLUBILITE DE LA DIHYDRO-4-TESTOSTERONE EN FONCTION DES CONCENTRATIONS D'HYDROXYPROPYLBETA-CYCLODEXTRINE DANS L'EAU.

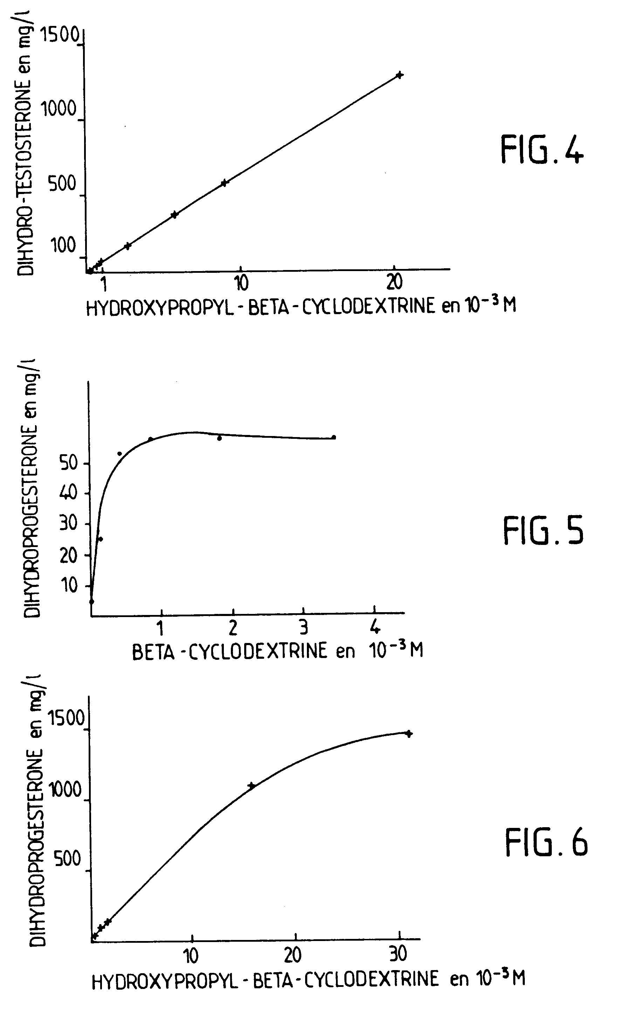

On travaille selon les mêmes conditions qu'à l'exemple 1. Les résultats obtenus sont schématisés par la courbe de la figure 4.

### EXEMPLE 5

### SOLUBILITE DE LA DIHYDRO-4-PROGESTERONE EN FONCTION DE LA CONCENTRATION DE LA BETACYCLODEXTRINE DANS L'EAU.

On suit encore le même protocole qu'à l'exemple 1 sauf en ce que la concentration en bétacyclodextrine varie de 0 à 4mM et les résultats font l'objet de la courbe de la figure 5.

### EXEMPLE 6

### SOLUBILITE DE LA DIHYDRO-4-PROGESTERONE EN FONCTION DE LA CONCENTRATION DE L'HYDROXYPROPYLBETA-CYCLODEXTRINE DANS L'EAU.

Ici encore on suit le même protocole qu'à l'exemple 1 sauf en ce que la concentration en hydroxypropybéta-cyclodextrine varie de 0 à 32mM et les résultats sont représentés par la courbe de la figure 6.

Les solubilités optimales des trois stéroïdes faisant l'objet des exemples précédents pour les concentrations adéquates de bétacyclodextrine et d'hydroxy-béta-cyclodextrine que l'on peut relever sur les courbes précédentes sont notées au tableau 2 suivant :

**TABLEAU 2**

| SOLUBILITE DES STEROIDES | | | |
|---|---|---|---|
| STEROIDES | SOLUBILITE EN GRAMMES PAR LITRE | | |
| | dans l'eau | dans eau + bétacyclodextrine | dans eau + hydroxypropylbétacyclodextrine |
| Prégnanolone | 0,005 | 0,44 | 3 |
| Dihydrotestostérone | 0,003 | 0,045 | 1,2 |
| Dihydroprogestérone | 0,005 | 0,06 | 1,4 |

### EXEMPLE 7

### PREPARATION D'UN COMPLEXE DE LA PREGNANE-5-BETA-OLE-3-ALPHA-ONE-20 ET COMPARAISONS.

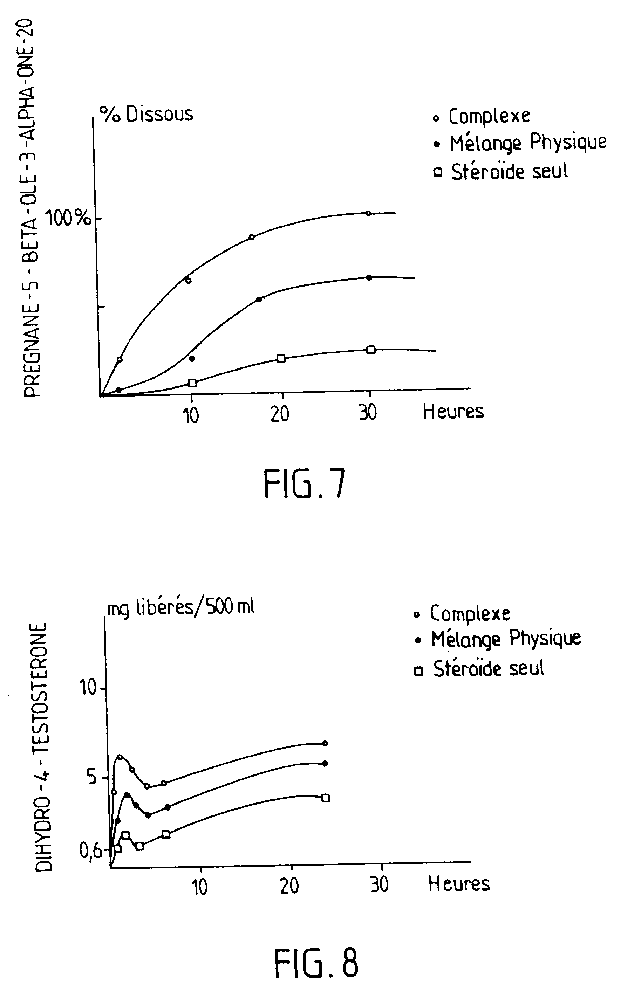

On dissout 0,5 gramme de bétacyclodextrine dans 50ml d'eau et 50mg de prégnane-5béta-ole-3alpha-one-20 dans 50ml d'éthanol à 95% V/V. On réunit les deux fractions que l'on place sous agitation magnétique pendant 30 minutes à environ 25°C. On évapore l'éthanol. Un précipité se forme. On place la fraction aqueuse restante et son précipité sous agitation magnétique pendant 24 heures à 25°C ± 1°C. On filtre la solution. On élimine l'eau ; on homogénéise la poudre ainsi obtenue. On effectue sur le complexe une dissolution après avoir dosé le taux de stéroïdes qui est de 5%. Les résultats sont illustrés par les courbes de la figure 7 qui permettent de comparer la solubilité de la prégnane-5béta-ole-3alpha-one-20 ou prégnanolone seule (courbe inférieure) (courbe intermédiaire) avec celle du simple mélange physique de ce stéroïde et de la bétacyclodextrine (courbe intermédiaire) et avec la solubilité du complexe stéroïde/bétacyclodextrine (courbe supérieure). On remarquera d'ailleurs que dès la mise en contact des constituants c'est-à-dire ce qui est nommé ici le mélange physique, avant même que ne se complète l'inclusion une première augmentation de la solubilité apparaît de façon extrêmement nette.

### EXEMPLE 8

### PREPARATION D'UN COMPLEXE DE LA DIHYDRO-4-TESTOSTERONE ET COMPARAISONS.

On suit le même protocole qu'à l'exemple 7 et on constate que le taux de stéroïdes est également de 5%. Les courbes de solubilité sont représentées à la figure 8 comme à la figure 7 la courbe inférieure représente la solubilité du stéroïde seul, c'est-à-dire de la dihydro-4-testostérone, la courbe intermédiaire correspond au mélange physique stéroïde + cyclodextrine avec la même remarque que précédemment, et la courbe supérieure représente la solubilité du complexe dihydro-4-testostérone/hydroxypropylbétacyclodextrine.

Il ressort de tous les exemples précédents que le choix très particulier d'une bétacyclodextrine, et en notamment de l'hydroxypropylbétacyclodextrine, convient tout particulièrement à la solubilisation des stéroïdes appartenant à la famille plus haut définie. Il en va d'ailleurs de même avec la gammacyclodextrine. Comme on vient de le remarquer la simple mise en présence physique des constituants augmente déjà la solubilité mais lorsque la complexation ou des phénomènes équivalents se produisent on obtient une augmentation considérable de la solubilité, puisque dans certains cas on a constaté une multiplication du taux de solubilité par un coefficient de plusieurs centaines (voir, par exemple, au tableau 2 la solubilité la dihydro-4-testostérone).

L'invention concerne les formes galéniques des produits ainsi obtenus et les applications thérapeutiques des stéroïdes actifs ainsi inclus.

## Revendications

1. Complexe résultant de l'inclusion d'un stéroïde dans une cyclodextrine, caractérisé par le fait que le stéroïde comprend de 18 à 27 atomes de carbone et répond à la formule suivante : 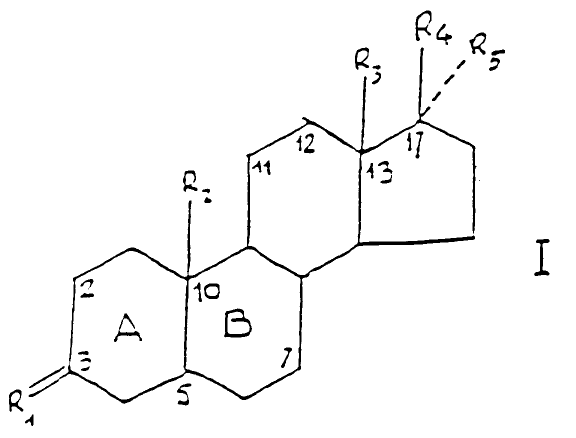 dans laquelle :
- les noyaux A et B sont saturés,
- R₁ est de l'hydrogène et/ou un radical alcool (-OH) ou cétone (=O ),
- l'un au plus de R₂ et R₃ est un hydrogène, l'un au moins de R₂ et R₃ étant un radical méthyle,
- R₄ est un hydrogène, un alcool ou une chaîne hydrocarbure alipathique éventuellement ramifiée et portant éventuellement une ou plusieurs fonctions alcool et/ou cétone et/ou acide;
- R₅ est un atome d'hydrogène ou un radical alcool ou un hydrocarbure aliphatique.

2. Complexe suivant la revendication 1 caractérisé en ce que la cyclodextrine est la bétacyclodextrine.

3. Complexe suivant la revendication 1 caractérisé en ce que la cyclodextrine est l'hydroxypropylcyclodextrine.

4. Complexe suivant la revendication 1 caractérisé en ce que la cyclodextrine est la gamma cyclodextrine.

5. Complexe suivant l'une quelconque des revendications 1 à 4 caractérisé en ce qu'au moins l'un de R₄ et R₅ de la formule est un radical cétone.

6. Complexe suivant l'une quelconque des revendications 1 à 5 caractérisé en ce qu'entre les positions 2 et 3 est formé un hétérocycle incluant les deux atomes de carbone desdites positions.

7. Complexe suivant la revendication 6 caractérisé en ce que l'hétérocycle est aminé et comprend au moins un atome d'azote.

8. Complexe suivant la revendication 6 ou la revendication 7 caractérisé en ce que l'hétérocycle comprend un atome d'oxygène.

9. Complexe suivant l'une quelconque des revendications précédentes caractérisé en ce qu'au moins un radical méthyle est fixé en position 1 et/ou 2.

10. Complexe suivant l'une quelconque des revendications précédentes caractérisé en ce qu'un radical acide est fixé en position 2.

11. Complexe suivant l'une quelconque des revendications précédentes caractérisé en ce qu'une fonction cétone et/ou une fonction alcool et/ou un hydrogène sont fixés en positions 7 et/ou 11 et/ou 12.

12. Procédé d'inclusion d'un stéroïde dans une cyclodextrine caractérisé par le fait que le stéroïde et la cyclodextrine sont choisis selon l'une quelconque des revendications 1 à 11 et mis en solution dans l'eau.

13. Procédé suivant la revendication 12 caractérisé en ce que la solution est hydroalcoolique.

14. Forme galénique caractérisée par le fait qu'elle résulte des produits et procédés selon l'une quelconque des revendications 1 à 13.

## Claims

1. Complex resulting from the inclusion of a steroid in a cyclodextrin, characterized in that the steroid comprises from 18 to 27 atoms of carbon and responds to the following formula: 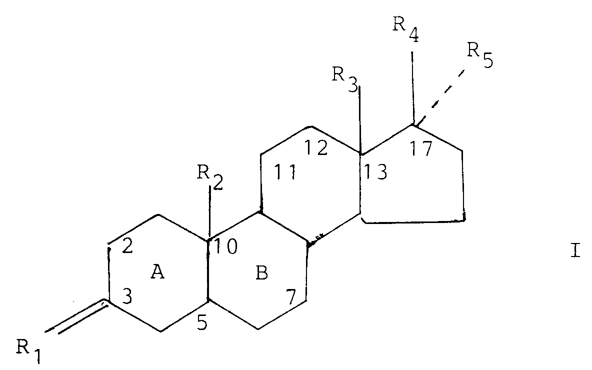 in which:
- the rings A and B are saturated,
- R₁ is hydrogen and/or an alcohol radical (-OH) or ketone (=O),
- at the most one of R₂ and R₃ is a hydrogen, at least one of R₂ and R₃ being a methyl radical,
- R₄ is a hydrogen, an alcohol or a possibly branched aliphatic hydrocarbon chain possibly bearing one or more alcohol and/or ketone and/or acid functions,
- R₅ is an atom of hydrogen or an alcohol radical or an aliphatic hydrocarbon.

2. Complex according to Claim 1, characterized in that the cyclodextrin is betacyclodextrin.

3. Complex according to Claim 1, characterized in that the cyclodextrin is hydroxypropylcyclodextrin.

4. Complex according to Claim 1, characterized in that the cyclodextrin is gamma cyclodextrin.

5. Complex according to any one of Claims 1 to 4, characterized in that at least one of R₄ and R₅ of the formula is a ketone radical.

6. Complex according to any one of Claims 1 to 5, characterized in that, between positions 2 and 3 there is formed a heterocycle including the two carbon atoms of said positions.

7. Complex according to Claim 6, characterized in that the heterocycle is animated and comprises at least one atom of nitrogen.

8. Complex according to Claim 6 or Claim 7, characterized in that the heterocycle comprises an atom of oxygen.

9. Complex according to any one of the preceding Claims, characterized in that at least one methyl radical is fixed in position 1 and/or 2.

10. Complex according to any one of preceding Claims, characterized in that an acid radical is fixed in position 2.

11. Complex according to any one of the preceding Claims, characterized in that a ketone function and/or an alcohol function and/or a hydrogen are fixed in positions 7 and/or 11 and/or 12.

12. Process for inclusion of a steroid in a cyclodextrin, characterized in that the steroid and the cyclodextrin are chosen according to any one of Claims 1 to 11 and placed in solution in water.

13. Process according to Claim 12, characterized in that the solution is water-alcohol.

14. Galenic form, characterized in that it results from the products and processes according to any one of Claims 1 to 13.

## Patentansprüche

1. Durch Einschluß eines Steroids in einem Cyclodextrin erhaltener Komplex, dadurch gekennzeichnet, daß das Steroid 18 bis 27 Kohlenstoffatome umfaßt und der nachfolgenden Formel entspricht:
I. 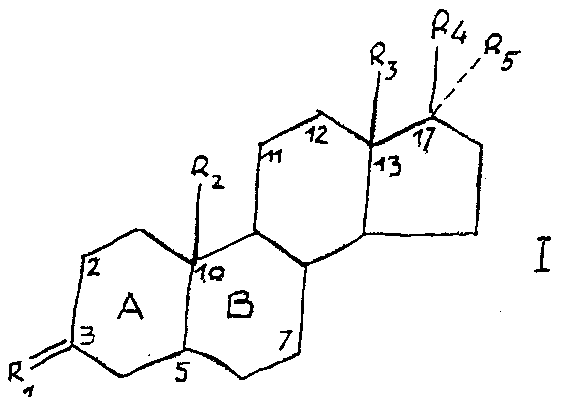 in der
A und B gesättigte Ringe sind,
R₁ Wasserstoff und/oder eine alkoholische (-OH) oder Kethongruppe (= O) ist, höchstens eine der Gruppen R₂ und
R₃ Wasserstoff und mindestens eine der Gruppen R₂ und R₃ eine Methyl-Gruppe ist,
R₄ Wasserstoff, ein Alkohol oder eine aliphatische Kohlenwasserstoffkette ist, die gegebenenfalls verzweigt ist und gegebenfalls eine oder mehrere alkoholische und/oder Keto- und/oder Säure-Funktionen aufweist;
R5 ein Wasserstoffatom oder ein alkoholisches Radikal oder eine aliphatische Kohlenwasserstoff-Gruppe ist.

2. Komplex nach Anspruch 1, dadurch gekennzeichnet, daß das Cyclodextrin β-Cyclodextrin ist.

3. Komplex nach Anspruch 1, dadurch gekennzeichnet, daß das Zyclodextrin Hydroxypropylcyclodextrin ist.

4. Komplex nach Anspruch 1, dadurch gekennzeichnet, daß das Cyclodextrin γ-Cyclodextrin ist.

5. Komplex nach einem der Ansprüche 1 bis 4, dadurch gekennzeichnet, daß mindestens eine der Gruppen R₄ und R₅ der Formel eine Keton-Gruppe ist.

6. Komplex nach einem der Ansprüche 1 bis 5, dadurch gekennzeichnet, daß zwischen den Positionen 2 und 3 ein Heterozyklus gebildet wird, der die beiden Kohlenstoffatome der genannten Positionen einschließt.

7. Komplex nach Anspruch 6, dadurch gekennzeichnet, daß der Heterozyklus aminiert ist und mindestens ein Stickstoffatom umfaßt.

8. Komplex nach Anspruch 6 oder 7, dadurch gekennzeichnet, daß der Heterozyklus ein Sauerstoffatom umfaßt.

9. Komplex nach einem der vorangehenden Ansprüche, dadurch gekennzeichnet, daß mindestens ein Methyl-Radikal in Position 1 und/oder 2 gebunden ist.

10. Komplex nach einem der vorangehenden Ansprüche, dadurch gekennzeichnet, daß eine Säure-Gruppe in Position 2 gebunden ist.

11. Komplex nach einem der vorangehenden Ansprüche, dadurch gekennzeichnet, daß eine Keton- und/oder eine alkoholische Gruppe und/oder ein Wasserstoffatom in den Positionen 7 und/oder 11 und/oder 12 gebunden sind.

12. Verfahren zum Einschluß eines Steroids in einem Cyclodextrin, dadurch gekennzeichnet, daß das Steroid und das Cyclodextrin gemäß einem der Ansprüche 1 bis 11 gewählt sind und in Wasser gelöst werden.

13. Verfahren nach Anspruch 12, dadurch gekennzeichnet, daß die Lösung wässrig/alkoholisch ist.

14. Galenische Form, dadurch gekennzeichnet, daß sie aus Produkten und Verfahren nach einem der Ansprüche 1 bis 13 erhalten wird.
